# FASCICULE DE BREVET EUROPEEN

(11) **EP 2 846 764 B1**
(45) Date de publication et mention de la délivrance du brevet: **04.07.2018**
(21) Numéro de dépôt: 13727262.1
(22) Date de dépôt: 06.05.2013
(51) Int. Cl.: A61K 8/34, A61K 8/73, A61K 8/81, A61Q 19/08

(54) **COMPOSITION ET PROCEDE DE SOIN COSMETIQUE UTILISANT UN MELANGE ELASTIQUE**
KOSMETISCHE PFLEGEZUSAMMENSETZUNG UND -VERFAHREN UNTER VERWENDUNG EINER ELASTISCHEN MISCHUNG
COSMETIC CARE COMPOSITION AND METHOD USING AN ELASTIC MIXTURE

(30) Priorité: 10.05.2012 FR 1254269
(43) Date de publication de la demande: 18.03.2015
(73) Titulaire: LvmH Recherche, 45800 Saint-Jean De Braye (FR)
(72) Inventeur: GOMBART, Emilie, F-45000 Orleans (FR); TRANCHANT, Jean-François, F-45760 Marigny Les Usages (FR); POULIN, Alex, F-45150 Jargeau (FR)
(74) Mandataire: Mena, Sandra
(86) Numéro de dépôt international: PCT/FR2013/051005
(87) Numéro de publication internationale: WO 2013/167835

(56) Documents cités:
- EP-A1- 0 970 681
- EP-A2- 1 892 015
- WO-A1-03/030882
- WO-A1-2008/012220
- WO-A2-00/56270
- "Monograph ID 115: Aluminium Starch Octenylsuccinate" In: "International Cosmetic Ingredient Dictionary and Handbook,11th edition", janvier 2006 (2006-01), CTFA, XP002725309, ISBN: 1882621360 vol. 1, page 112, Monograph ID 115

## Description

La présente invention se rapporte à un procédé de soin cosmétique qui consiste à appliquer sur la peau une composition comprenant un mélange apte à former un film élastique. L'invention a également pour objet une composition particulière contenant ce mélange élastique. La composition est de bonne tenue sur la peau, et procure un film confortable, souple et élastique. La composition procure un effet de tension de la peau, et se déforme élastiquement pour suivre les mouvements et les déformations mécaniques de la peau, notamment la peau du visage.

La demanderesse a décrit dans la demande WO 2003/017967 un mélange tenseur de la surface de la peau comprenant 10 à 80% en poids d'un alginate, de 15 à 75% en poids d'un sucre tel que le sorbitol, et de 3 à 15% en poids d'alcool polyvinylique ou de polyvinylpyrrolidone. L'introduction d'alcool polyvinylique ou de polyvinylpyrrolidone dans des mélanges contenant un polysaccharide et un sucre permet d'améliorer les propriétés d'adhésion à la peau. En outre, l'addition de cellulose à ce mélange ternaire permet d'éviter toute sensation de tiraillement de la peau, en conférant de l'élasticité à la matrice polymérique formée après application de la composition sur la peau, sans provoquer pour autant un relâchement. Le document EP 0970681 décrit un mélange tenseur de la surface de la peau comprenant 23% en poids d'une dextrine,environ 58% en poids d'alcool polyvinylique et environ 19% en poids de glycérol. Il a été trouvé de manière tout à fait inattendue, dans le cadre d'une nouvelle étude, que l'introduction d'amidon ou d'un de ses dérivés, dans un film de polymère plastifié permet de lui conférer une grande élasticité, et qu'un tel mélange peut être incorporé dans une composition cosmétique qui demeure stable et homogène. Les films obtenus à partir de ce mélange ont une élasticité au moins dix fois supérieure à celle du mélange tenseur décrit dans la demande WO 2003/017967.

La présente invention fournit ainsi un nouveau procédé de soin cosmétique qui consiste à appliquer sur une partie du corps, notamment la peau du visage ou du décolleté, un produit cosmétique comprenant un mélange d'excipients qui procure de l'élasticité à la peau naturellement sollicitée par des déformations, telles que les changements d'expression du visage ou le contact avec un vêtement ou les mains. Ce mélange d'excipients permet avantageusement d'obtenir sur la zone du corps concernée, dans certains modes de mise en oeuvre, un film transparent et doux au toucher, de bonne tenue au cours du temps et sans être trop collant. De plus, le film peut se déformer réversiblement, de sorte qu'il reprend après déformation, par exemple après étirement, l'état qu'il avait avant l'application de la contrainte mécanique fournie par exemple par une déformation de la peau. Le film que forme la composition comprenant ledit mélange a une plus grande résistance à la cassure, ce qui permet de maintenir intactes les propriétés mécaniques du film formé à la surface de la peau, après application et séchage éventuel de la composition.

L'invention a donc pour objet un procédé de soin cosmétique qui consiste à appliquer sur au moins une partie du corps, une composition cosmétique contenant de l'eau et un mélange apte à former un film élastique constitué
- d'au moins un amidon gélifiant ou épaississant,
- d'au moins un polymère choisi parmi les alcools polyvinyliques, les polymères et copolymères de vinylpyrrolidone et
- d'au moins un polyol, et
- éventuellement d'eau.

Par « amidon » au sens de l'invention, on entend un hydrolysat d'amidon. Le procédé de l'invention est avantageusement caractérisé en ce qu'on applique ladite composition sur au moins une zone de peau, en particulier la peau du visage ou du décolleté, laquelle présente des signes de vieillissement ou de fatigue tels qu'une perte de fermeté, une perte d'élasticité ou un relâchement cutané, pour obtenir un effet choisi parmi un effet lissant, un effet tenseur, un effet rebondi de la peau, ou une association de l'un de ces effets.

L'invention a pour autre objet une composition cosmétique contenant un mélange apte à former un film élastique constitué d'au moins un hydrolysat d'amidon, d'au moins un polyol, d'au moins un polymère choisi parmi les alcools polyvinyliques, les polymères et copolymères de vinylpyrrolidone et éventuellement d'eau.
La composition de l'invention est avantageusement dépourvue de dérivés de celluloses, comme l'hydroxypropylcellulose, l'hydroxyméthylcellulose, l'hydroxyéthylcellulose, l'hydroxypropylméthylcellulose ou la carboxyméthylcellulose. La composition de l'invention peut être dépourvue d'un acide faible de pKa supérieur à 2. La composition contient de préférence moins de 1% en poids d'un alcool inférieur tel que l'éthanol ou l'isopropanol.

Dans la description qui suit, le terme « composition cosmétique » se réfère aussi bien à la composition cosmétique selon l'invention qu'à la composition cosmétique utilisée dans le procédé de l'invention. Dans la description, on parle indifféremment de mélange élastique ou de mélange apte à former un film élastique. La composition cosmétique contient entre 0,2 et 90 % en poids d'un mélange apte à former un film élastique constitué d'au moins un amidon gélifiant ou épaississant, d'au moins un polyol et d'au moins un polymère choisi les alcools polyvinyliques, les polymères et copolymères de vinylpyrrolidone dans lequel l'amidon gélifiant ou épaississant représente entre 10 et 20% en poids sec, le polymère représente de 35 à 45% en poids sec, et le polyol représente entre 35 et 45% en poids sec, par rapport au poids du mélange, la somme de ces trois pourcentages étant égale à 100.

L'expression « en poids sec » entend désigner le poids du composé dépourvu d'eau. L'expression « comprise entre ... et... » entend exclure les bornes. L'expression « de ... à .... » entend inclure les bornes.

Dans le mélange élastique, l'amidon, le polymère et le polyol sont de préférence dans des proportions telles que l'élasticité d'un film sec (dépourvu d'eau), d'épaisseur comprise entre 50 et 1000 microns, obtenu à partir de ce mélange est caractérisée par
- une élongation à la rupture, encore appelée pourcentage de déformation à la rupture, supérieure à 50%, et
- un taux de déformation résiduelle, mesuré après au moins une traction, inférieur à 15%.

L'élasticité du mélange peut être mesurée par une méthode bien connue de l'homme du métier, par exemple avec un Texturomètre de référence TA XT Plus, par mesure de la force exercée par un film sec obtenu à partir du mélange et subissant un essai en traction à vitesse imposée. L'épaisseur du film choisi pour réaliser cette mesure est de préférence comprise entre 50 et 1000 microns, de préférence encore entre 90 et 110 microns.

La nature chimique du polyol ainsi que les quantités de polyol et d'amidon sont de préférence choisies de manière à ce que le mélange procure une sensation agréable au toucher, sans être trop collant, tout en assurant une bonne adhésion sur la peau. En fonction du type de composition cosmétique que l'on souhaite préparer, on pourra définir un compromis entre les propriétés de collant et d'adhésion procurées par le mélange, de manière à ce que le produit cosmétique vérifie les propriétés attendues.

Selon un mode de mise en oeuvre, le mélange élastique est constitué d'au moins un hydrolysat d'amidon, d'au moins un polymère choisi parmi les alcools polyvinyliques, les polymères et copolymères de vinylpyrrolidone et les latex, et d'au moins un polyol, et éventuellement d'eau.

Le mélange est avantageusement dépourvu d'un alginate, ou de tout autre ingrédient qui risquerait de déstabiliser le mélange du polymère, du polyol et de l'amidon.

L'amidon est épaississant ou gélifiant, au sens où il est susceptible de produire dans l'eau ou dans une phase aqueuse, des solutions, des suspensions ou des gels visqueux colloïdaux.
Par « amidon » au sens de l'invention, on entend un hydrolysat d'amidon. On entend par amidon « natif », un amidon qui n'a pas subi de modification de sa structure chimique. On entend par « dérivé » d'amidon, un amidon natif qui a subi des traitements chimiques, enzymatiques ou thermiques, doté de propriétés gélifiantes et épaississantes. Par exemple, le dérivé d'amidon de nom INCI Aluminium Starch Octenyl Succinate n'est pas un amidon qui gélifie ou épaissie de l'eau. Ce dérivé d'amidon est généralement utilisé dans des compositions cosmétiques comme absorbant d'huile ou de sébum, ou comme anti-perspirant. De même, les amidons généralement utilisés à titre de charge dans les compostions cosmétiques ne sont pas des amidons gélifiants au sens de la présente invention, car ils ne sont pas présents dans une phase aqueuse et n'interagissent pas avec l'eau présente dans la composition pour la gélifier ou l'épaissir. De même, l'amidon de maïs estérifié (Dry Flow Plus®) n'est pas un amidon gélifiant ou épaississant.

Les amidons peuvent subir des modifications chimiques telles que par exemple une acétylation, dans un degré tel que les propriétés gélifiantes et épaississantes de l'amidon sont conservées.

Les amidons utilisés dans le cadre de la présente invention présentent l'avantage de procurer en mélange avec les excipients précités des films secs élastiques qui sont transparents et qui restent transparents après étirement(s) lorsqu'ils sont préparés sous forme de films fins (typiquement d'épaisseurs inférieures au millimètre). Avec d'autres polymères que l'amidon, le film sec peut être élastique et transparent mais perdre sa transparence après avoir subi des déformations mécaniques. Il est particulièrement avantageux dans le cadre de la présente invention que le film formé sur la peau par le mélange conserve sa transparence sous l'effet de multiples sollicitations mécaniques, de manière à ce qu'il ne perturbe pas au cours du temps la couleur du dépôt sur la peau de la composition cosmétique dans laquelle le mélange a été introduit.

L'amidon est choisi parmi les hydrolysats d'amidon. Parmi les hydrolysats d'amidon, on peut citer les dextrines et les maltodextrines. Les dextrines peuvent porter le numéro CAS 9004-53-9.

Les hydrolysats d'amidon sont classés selon leur apport en dextrose encore appelé dextrose équivalent DE. Le DE est le nombre de grammes de sucres réducteurs (considérés comme du dextrose) pour 100 g de matières sèches du produit. Les dextrines au sens de l'invention sont des hydrolysats d'amidon dont le DE est notamment compris entre 1 et 13, et les maltodextrines sont des hydrolysats d'amidon dont le DE est notamment compris entre 3 et 20.
Les dextrines et maltodextrines utilisées selon l'invention peuvent être obtenues par l'hydrolyse partielle acide et/ou enzymatique d'un amidon natif. Divers procédés d'hydrolyse sont connus. L'amidon subissant une hydrolyse peut provenir d'une origine variée mais de préférence du maïs, de fécule de pomme de terre, du tapioca, du riz ou du manioc.
Les dextrines ou maltodextrines utilisables selon l'invention se présentent sous forme de poudre blanche, jaune ou brune ou d'une solution aqueuse concentrée.

Selon un mode de mise en oeuvre préféré, le polymère est un alcool polyvinylique.
Un alcool polyvinylique comporte des unités -(CH₂-CHOH)- et éventuellement des unités -(CH₂-CH(OCOCH₃))- en une quantité maximum de 5% en mole par rapport au polymère final.
De préférence, les unités -(CH₂-CH(OCOCH₃))- sont présentes dans le polymère en une quantité de 0 à 3% en mole, notamment en une quantité de 0,05 à 2% en mole. Une quantité de 5% en mole par rapport au polymère final d'unités - (CH₂-CH(OCOCH₃))- correspond à un degré d'hydrolyse de l'alcool polyvinylique de 95%.
De préférence, l'alcool polyvinylique a un poids moléculaire moyen en masse (Mw) compris entre 30 000 et 500 000 g/mole, notamment de 50 à 200 000 g/mole, et encore mieux de 80 000 à 150 000 g/mole.
Comme alcool polyvinylique utilisable selon l'invention, on peut notamment citer ceux vendus sous les dénominations CELVOL®, et plus particulièrement le CELVOL® 523.

Le polyol a avantageusement un faible poids moléculaire, inférieur à celui de l'alcool polyvinylique, par exemple en poids moléculaire (Mw) inférieur ou égal à 500 g/mole.
Les polyols particulièrement préférés sont les alcools ayant de 1 à 18 atomes de carbone et 2 à 6 fonctions hydroxyles, notamment ceux ayant de 2 à 12 atomes de carbone et/ou 2 à 4 fonctions hydroxyles.

Le polyol peut être choisi parmi les sucres et leurs dérivés, en particulier leurs esters ou leurs éthers. Le sucre est par exemple choisi parmi les sucres en C₆ et les sucres en C₁₂. Un sucre en C₆ peut être le glucose, le sorbitol, le mannitol ou le galactitol. Un sucre en C₁₂ peut être le saccharose ou le lactitol. Le polyol peut être choisi parmi les polyalkylène-glycols, notamment les poly(oxyalkylène en C₂-C₅) et plus particulièrement un poly(oxyde d'éthylène) et/ou un poly(oxyde de propylène).

Le polyol est de préférence choisi parmi le glycérol, le sorbitol et les glycols. Selon un mode de réalisation avantageux, le polyol est le glycérol.

On choisira de préférence la nature du polyol et sa quantité de manière ce que le polyol fasse gonfler l'amidon. Avantageusement, le polyol aide à la dispersion et plastifie le polymère.
Le ratio massique entre le polymère et le polyol est de préférence compris entre 30/70 et 70/30, de préférence entre 40/60 et 60/40, et de manière encore préférée entre 45/55 et 55/45.

Les mélanges élastiques préférés utilisés dans le procédé ou la composition de l'invention sont constitués de glycérol, d'alcool polyvinylique et de dextrine dans les proportions suivantes
- de 10 à 20 %, en poids de dextrine,
- de 35 à 45 % en poids d'alcool polyvinylique,
- de 35 à 45 %, en poids de glycérol,
ces pourcentages étant exprimés en poids sec de chacun des trois ingrédients par rapport au poids total des trois ingrédients, et la somme de ces trois pourcentages étant égale à 100.

Le mélange élastique peut permettre l'obtention d'un film ayant un pourcentage d'élongation à la rupture supérieure à environ 50%, de préférence supérieure à 100%, de préférence encore supérieure à 200 % et, de manière encore préférentielle, supérieure à 250 %. Le taux de déformation résiduelle d'un film du mélange élastique est avantageusement inférieur à 15%.
Le pourcentage d'élongation à la rupture et le taux de déformation résiduelle sont de préférence mesurés sur le mélange décrit précédemment qui se présente sous la forme d'un film, de préférence de forme rectangulaire ou carrée, et de préférence d'épaisseur au moins égale à 100 microns, et pouvant aller jusqu'à 1 000 microns.

Le pourcentage d'élongation à la rupture et le taux de déformation résiduelle sont de préférence mesurés par traction d'un échantillon du mélange sec.

L'essai en traction consiste à appliquer deux forces opposées sur l'échantillon. Les extrémités d'un échantillon de film sont serrées entre deux mâchoires de la machine d'essai en traction, l'une étant fixe et l'autre étant solidaire d'un piston pneumatique. L'échantillon est ensuite soumis à un étirement uniaxial à vitesse imposée sur une distance donnée. Les forces sont enregistrées par le capteur de force situé sur la partie fixe de la machine, et l'on trace un diagramme reproduisant la force exercée par l'échantillon en fonction de l'allongement de l'échantillon, ainsi qu'un diagramme reproduisant la force exercée par l'échantillon en fonction du temps.

Le taux de déformation résiduelle est de préférence mesuré après au moins une étape de traction, à une distance imposée égale à un tiers de la longueur de l'échantillon, par exemple à une distance allant de un tiers à quatre fois sa longueur. On peut également réaliser des cycles successifs d'étirements en augmentant graduellement la distance d'étirement.
Un tel film est essentiellement anhydre, c'est-à-dire qu'il comprend moins de 5% en poids d'eau.

Un tel film essentiellement anhydre comprend au moins 80% en poids du mélange, le solde étant constitué d'un ou plusieurs adjuvants et d'eau résiduelle. On entend par « film essentiellement anhydre », un film comprenant moins de 5% en poids d'eau résiduelle.
Les adjuvants peuvent être par exemple des colorants ou des parfums.

De tels films élastiques peuvent être par exemple utilisés comme support de tatouages éphémères ou décalcomanie, dont le motif, préalablement fixé au film, est transféré sur la peau par application du film comprenant le motif.
Dans cette application, le support, par exemple la peau, peut avoir été préalablement humecté d'eau pour faciliter le transfert. Le film est obtenu par étalement d'une composition aqueuse comprenant le mélange élastique sur un support et d'éventuels adjuvants, puis séchage. L'étalement est avantageusement réalisé de telle sorte que le film présente une épaisseur après séchage, qui soit identique sur toute sa surface.

Un autre objet de l'invention porte sur une l'utilisation du mélange qui vient d'être décrit pour conférer des propriétés élastiques à une composition cosmétique.

Le mélange de l'invention est incorporé dans ladite composition cosmétique à une concentration allant par exemple de 0,4 à 85 %, de préférence de 1 à 80 % en poids par rapport au poids total de ladite composition. Selon un mode de réalisation, le mélange de l'invention est incorporé dans ladite composition cosmétique à une concentration allant de 3 à 70%, par exemple de 5 à 50%, en poids par rapport au poids total de ladite composition.
Selon un autre mode de réalisation, le mélange de l'invention est incorporé dans ladite composition cosmétique à une concentration allant de 10 à 40%, voire de 20 à 30% en poids par rapport au poids total de ladite composition.

Dans un mode de réalisation particulier, la composition est sous la forme d'une émulsion huile-dans-eau contenant de 5 à 10 % en poids par rapport au poids total de ladite composition, du mélange élastique.

La quantité du mélange introduite dans la composition cosmétique est avantageusement choisie en fonction de l'effet cosmétique recherché et de la viscosité du produit obtenue.
La quantité du mélange est notamment choisie pour permettre au moment de l'application de la composition sur la peau, la formation d'un film élastique au moment du séchage de ladite composition.
La viscosité du produit doit être compatible avec une application sur la peau régulière, agréable et suffisante.

Les proportions entre les différents constituants seront optimisées pour avoir la bonne visco-élasticité et la bonne adhérence sur la peau. Suivant les formules, par exemple s'il s'agit d'un fond de teint ou d'un sérum, on cherchera un compromis entre l'adhérence sur la peau et la viscoélasticité de la composition. Par exemple, si la formule contient des poudres, la quantité de polyol à introduire sera plus importante afin de maintenir la plasticité et l'élasticité.

La composition cosmétique peut contenir au moins un polymère choisi les alcools polyvinyliques, les polymères et copolymères de vinylpyrrolidone lequel polymère est dans une proportion massique comprise de préférence entre 0,10 et 40%, entre 0,10 et 30%, entre 0,5 et 25%, entre 1,75 et 12%, entre 2 et 7%, entre 2,25 et 6,75%, entre 2,75 et 5,25, ou encore entre 3 et 3,5% en poids.

La composition cosmétique peut contenir au moins un polyol dans une proportion massique comprise de préférence entre 0,10 et 40%, entre 0,10 et 30%, entre 0,5 et 25%, entre 1,75 et 20%, entre 2 et 15%, entre 5 et 15%, ou encore entre 10 et 15% en poids.

La composition cosmétique peut contenir au moins un amidon dans une proportion massique comprise de préférence entre 0,03 et 17,5%, entre 0,05 et 13,5%, entre 0,25 et 10,5%, entre 0,75 et 5%, entre 0,8 et 4,5%, entre 1 et 3,75%, ou encore entre 1,25 et 2,25% en poids.

Selon un mode de mise en oeuvre, l'amidon représente de 0,05 à 5% en poids sec du poids de la composition, le polymère représente de 0,1 à 10% en poids du poids de la composition, et le polyol représente de 0,1 à 10% en poids du poids de la composition.

La composition cosmétique peut comprendre par ailleurs une phase aqueuse, qui peut comprendre, outre de l'eau, une eau florale telle que l'eau de bleuet, une eau minérale et/ou une eau thermale. De préférence, la composition comprend de 50 à 99% en poids, de préférence de 60 à 90% en poids, et de préférence encore de 70 à 80% en poids, par rapport au poids total de la composition, d'eau ou d'une phase aqueuse.

Suivant une variante de l'invention, ladite composition est caractérisée en ce qu'elle contient en outre au moins un actif cosmétique.

L'actif cosmétique peut en particulier être avantageusement choisi parmi un agent hydratant, un agent anti-ride, un agent anti-oxydant, un agent anti-radicalaire, un agent réparateur des effets destructeurs des rayons ultraviolets ou un agent amincissant tel que la caféine. L'agent hydratant est de préférence différent de l'urée.

L'invention concerne ainsi selon un de ses aspects une composition contenant de l'eau, au moins un actif cosmétique en des proportions variant de 0,01 à 5% en poids, et de 0,2 à 90% en poids sec du mélange décrit précédemment.

L'invention a encore pour objet une composition cosmétique contenant un mélange apte à former un film élastique constitué d'au moins un hydrolysat d'amidon, d'au moins un polyol, d'au moins un polymère choisi parmi les alcools polyvinyliques, les polymères et copolymères de vinylpyrrolidone et éventuellement d'eau.

Dans la description qui suit, le terme « composition cosmétique » se réfère aussi bien à la composition cosmétique selon l'invention qu'à la composition cosmétique utilisée dans le procédé de l'invention.

Dans la composition, l'amidon, par exemple l'hydrolysat d'amidon, représente avantageusement de 0,05 à 5% en poids du poids de la composition, le polymère, par exemple l'alcool polyvinylique, représente de préférence de 0,1 à 10% en poids du poids de la composition, et le polyol représente de préférence de 0,1 à 10% en poids du poids de la composition.

Suivant un mode préféré de réalisation de cette variante de l'invention, ladite composition est caractérisée en ce qu'elle contient en outre un parfum, une matière colorante cosmétiquement acceptable et/ou un agent protecteur des rayons ultraviolets UVA et UVB, notamment un filtre ou un nano-pigment, tel qu'un oxyde de zinc ou de titane.

Selon divers modes de réalisation particuliers, la composition cosmétique peut se présenter sous la forme d'un gel, d'une lotion, d'un sérum, d'une suspension, d'une émulsion huile-dans-eau, d'une émulsion eau-dans-huile, ou d'un masque.

Selon un premier mode de réalisation, la composition forme après étalement sur une surface plane et l'évaporation progressive de l'eau de la composition, un film comprenant des actifs cosmétiques, qui présente des propriétés mécaniques de type élastique et des propriétés d'adhésion sur la peau.
Un tel film est avantageusement essentiellement anhydre, c'est-à-dire qu'il comprend avantageusement moins de 5% en poids d'eau résiduelle, de préférence moins de 2% en poids d'eau résiduelle.

Selon ce mode de réalisation particulier, la composition peut être un masque de soin anhydre (contenant moins de 1 à 3% en poids d'eau) adapté à une application sur la peau du corps.
Un tel masque se présente sous la forme d'un film que l'on peut manipuler avec les doigts ou même découpé avant de l'appliquer sur la peau. Le film peut alors être préparé par dissolution du polymère, du polyol et de l'amidon dans de l'eau, et ajout éventuel d'actifs cosmétiques ou d'adjuvants cosmétiques (colorants, parfums...).
Le film ainsi obtenu peut être avantageusement découpé en bandes ou selon des formes adaptées à une application sur la peau du corps, par exemple le décolleté, ou sur une partie ou la totalité du visage.
Un masque de soin peut être ainsi appliqué sur la peau pendant un temps suffisant pour que diffusent les actifs cosmétiques du film vers les couches de la peau.

Dans ces applications, l'épaisseur du film est de l'ordre de 500 microns à 1 mm, compatible avec un étirement manuel du film en vue de son application sur la peau du corps, par exemple le décolleté, et/ou du visage.

Selon une autre alternative, la composition est un film élastique sensiblement exempt d'eau comprenant au moins 80% en poids du mélange d'excipients précités, le solde étant constitué d'agents actifs cosmétiques et éventuellement d'excipients cosmétiques.

Avantageusement le film préparé par séchage d'une composition aqueuse comprenant le mélange décrit précédemment, au moins un agent cosmétique et éventuellement d'autres excipients cosmétiques.
Le terme « sensiblement exempt d'eau » signifie que le film sec comprend moins de 5% en poids d'eau, de préférence moins de 2% en poids d'eau résiduelle.
Le film est avantageusement préparé par étalement de ladite composition aqueuse sur une plaque adaptée, puis par séchage de ladite composition avantageusement réalisé par induction.

Le film ainsi obtenu peut être découpé en bandes ou celui des formes adaptées à une application sur la peau du corps, par exemple le décolleté, ou sur une partie ou la totalité du visage.

Selon un autre mode de réalisation, la composition est une composition cosmétique appliquée directement sur la peau, qui forme sur ladite peau, après son application et l'évaporation progressive de l'eau de la composition, une pellicule qui présente des propriétés mécaniques de type élastique avec une texture qui se prête particulièrement bien au maquillage. De plus ce type de texture possède des propriétés utiles tant sur le plan esthétique que sur celui du confort d'utilisation notamment à proximité de zones fortement mobiles comme par exemple les yeux et les lèvres.
Les propriétés élastiques de la composition cosmétique sont particulièrement utiles pour une application sur des zones du corps fortement sollicités par des mouvements tels que la peau du visage.

Un autre objet de l'invention porte sur le procédé de préparation de la composition décrite précédemment.

Selon une première mise en oeuvre, le procédé de préparation de la composition comprend :
- une première étape dans laquelle on prépare le mélange apte à former un film élastique par dissolution préalable du polyol dans de l'eau, puis du polymère dans l'eau, puis de l'amidon gélifiant ou épaississant dans l'eau,
- une deuxième étape dans laquelle on disperse la mélange obtenu à la première étape dans une phase aqueuse comprenant des excipients cosmétiques.
Le mélange élastique peut être préparé par dissolution préalable du polyol, de préférence du glycérol, dans de l'eau. Le polymère est ensuite dissous ou dispersé dans le mélange précédent, avec un apport éventuel de chaleur et d'agitation. Une fois le polymère dissous ou dispersé, on ajoute l'amidon et l'on procède de préférence à une agitation à une température supérieure à 60°C, par exemple de l'ordre de 70 à 80°C pour bien homogénéiser les ingrédients entre eux.
Selon une deuxième étape, le mélange ainsi préparé dans l'eau est ajouté à une phase aqueuse comprenant des agents actifs cosmétiques et/ou d'autres excipients cosmétiques.

Selon un autre mode de mise en oeuvre, la composition est préparée en ajoutant successivement chacun des composants du mélange directement dans la phase aqueuse de la composition cosmétique portée à la température adéquate, sans mélange préalable.

Dans le cas particulier d'une émulsion, les composants du mélange sont ajoutés à la phase aqueuse dispersante avant dispersion de la phase grasse.

Selon une variante du procédé de l'invention, le mélange élastique décrit précédemment est dispersé de façon extemporanée dans une base cosmétique contenant de l'eau telle qu'une crème ou un sérum. La composition est ensuite appliquée sur la peau. L'invention concerne également un kit contenant le mélange élastique décrit précédemment, et une base cosmétique, conditionnés de façon séparée.

L'invention a encore pour objet l'utilisation d'un hydrolysat d'amidon pour augmenter l'élasticité d'un film d'alcool polyvinylique plastifié. L'amidon améliore en particulier l'élasticité, la tenue et le confort sur les matières kératiniques de la composition cosmétique.

Selon un autre de ses aspects, l'invention concerne l'utilisation du mélange tel que défini précédemment pour la préparation d'une composition cosmétique en vue d'obtenir sur la peau la formation d'un film élastique, procurant un effet de tension de la peau après séchage de la composition.

Selon un autre de ses aspects, l'invention a pour objet un procédé de soin cosmétique qui consiste à appliquer sur au moins une partie du corps, une composition cosmétique contenant de l'eau et un mélange apte à former un film élastique constitué d'au moins un amidon gélifiant ou épaississant, d'au moins un polymère choisi parmi les alcools polyvinyliques, les polymères et copolymères de vinylpyrrolidone et les latex, et d'au moins un polyol, le mélange étant tel que
- l'amidon représente entre 10 et 20% en poids sec,
- le polymère représente de 35 à 45% en poids sec, et
- le polyol représente entre 35 à 45% en poids sec, par rapport au poids du mélange, la somme de ces trois pourcentages étant égale à 100.

Les caractéristiques qui ont été décrites précédemment en relation avec la composition cosmétique s'appliquent au procédé de l'invention.

Selon le procédé de soin cosmétique de l'invention, on peut appliquer la composition sur au moins une zone de peau, en particulier la peau du visage ou du décolleté, présentant des signes de vieillissement ou de fatigue tels qu'une perte de fermeté, une perte d'élasticité, ou un relâchement cutané, pour obtenir un effet choisi parmi un effet lissant, un effet tenseur, un effet rebondi de la peau, ou une association de l'un de ces effets.

Le procédé de soin cosmétique peut comprendre l'application topique sur les zones de la peau concernées d'une quantité efficace d'une composition cosmétique telle que définie précédemment, pour obtenir une sensation de seconde peau.

Dans les exemples, tous les pourcentages sont donnés en poids, la température est la température ambiante, la température est donnée en degrés Celsius et la pression est la pression atmosphérique, sauf indication contraire.

### Exemple 1 : Mesures d'élasticité du mélange élastique utilisé dans le procédé de l'invention

On a préparé des films constitués d'un mélange élastique selon l'invention ainsi qu'un mélange tenseur décrit dans la demande WO 2003/017967. Leur composition est donnée en pourcentages massiques des ingrédients désignés par leur dénomination INCI.

### Mélange tenseur comparatif

| INCI ou nom chimique | |
|---|---|
| Sorbitol | 56,1 |
| Algin | 28,0 |
| Alcool polyvinylique | 11,3 |
| Cellulose gum | 4,6 |

### Mélange élastique

| INCI ou nom chimique | |
|---|---|
| Glycérol | 40 |
| Dextrine | 20 |
| Alcool polyvinylique (PVA) | 40 |

### Préparation des échantillons :

On a préparé une solution aqueuse contenant 8% du mélange élastique en suivant le protocole qui suit.
L'eau et le glycérol ont été mélangés à 25°C dans un agitateur Rayneri (défloculeuse), puis l'amidon a été ajouté en pluie fine. Le mélange intermédiaire ainsi obtenu a été maintenu sous agitation Rayneri assez forte dans le but de créer un vortex, à une température de 70-80°C au bain-marie.
Le PVA a été ensuite ajouté en pluie fine toujours sous agitation et à 70°C. Le tout été mélangé pendant 30 minutes sous agitation. Si le mélange comprenait des bulles, il a été centrifugé à 2000 tr/min pendant 15 min.

On a préparé une solution aqueuse contenant 8% du mélange tenseur comparatif en suivant l'enseignement de la demande WO 2003/017967.

Chaque solution a été étalée sur une plaque de PMMA avec un barreau étaleur calibré pour obtenir un film d'épaisseur contrôlée de 700 µm. Les films ont été mis à sécher pour que l'eau qu'ils contiennent s'évapore.

On a découpé des éprouvettes rectangulaires de 50 mm de long et 30 mm de large.

### Essais en traction

Les propriétés mécaniques des éprouvettes séchées ont été évaluées à l'aide d'une machine d'essai en traction de référence Texturomètre TA XT® plus.

L'appareil a été calibré en distance et en force avant de réaliser les mesures. Pour cela, l'entrefer entre les deux étaux a été réglé à 30 mm, et la pré-tension des échantillons a été imposée avec une force de traction initiale de 20 g, pour compenser l'allongement du film dû au serrage de celui-ci dans les étaux.

Les échantillons ont été soumis à une traction uniaxiale (vitesse constante de 1 mm/s). Les informations fournies par l'enregistreur ont été la force exercée par l'échantillon exprimée en Newton et l'allongement de l'échantillon en millimètre. L'appareil a enregistré la force exercée par l'échantillon en fonction de son étirement ainsi que la force exercée par l'échantillon en fonction du temps.
A partir de ces informations, ont été calculés :
1. le pourcentage de la déformation du film au point de rupture, égale au rapport entre la longueur de l'échantillon au point de rupture et la longueur de l'échantillon avant toute traction (on entend par longueur de l'échantillon la distance entre les entrefers), et
2. le pourcentage de déformation persistante (que l'on peut encore appelée permanente, définitive ou résiduelle) de l'échantillon après plusieurs cycles d'étirements, exprimée comme le rapport entre i) la différence entre la longueur de l'échantillon après les étirements et la longueur initiale, et ii) la longueur initiale.

On peut réaliser les cycles d'étirement suivants:
a) trois cycles d'étirements correspondants chacun à une traction de 10 mm suivie d'un retour à 0 mm, puis trois cycles de 20 mm, puis trois cycles de 30 mm, ou
b) deux cycles de 60 mm, deux cycles de 90 mm, puis deux cycles de 120 mm,
c) deux cycles de 120 mm, ou
d) la combinaison des séquences a et b, ou
e) la combinaison des séquences a et c.
On entend par « cycle » un étirement de l'échantillon à la vitesse imposée, suivie d'un retour à la position initiale à la même vitesse. La distance initiale entre les entrefers est égale à 30 mm.

Deux échantillons ont été testés en traction successivement pour vérifier la reproductibilité des résultats en appliquant la séquence d'étirements a).

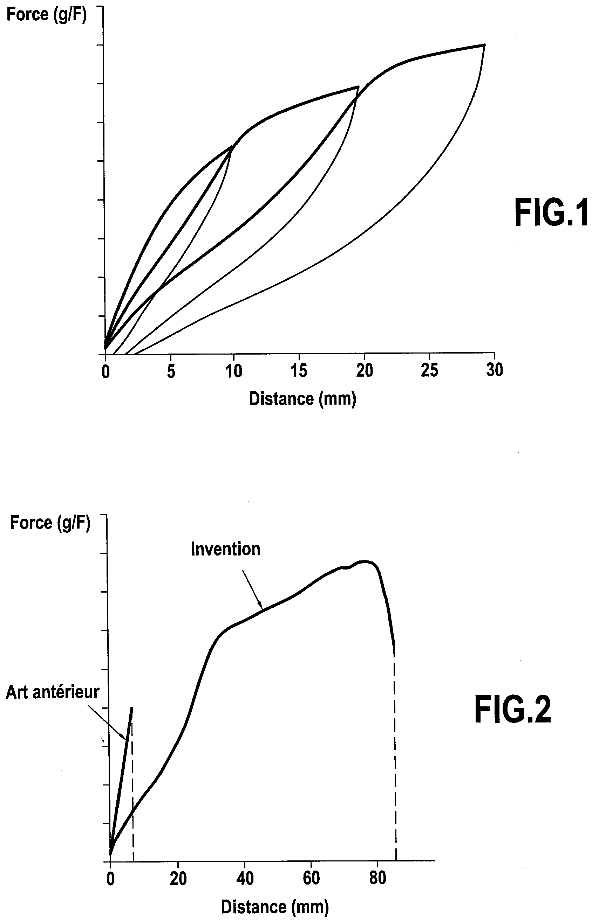

La déformation résiduelle du mélange élastique est égale à 9-10 % après trois étirements de 10 mm, suivis de trois étirements de 20 mm, suivis de trois étirements de 30 mm. Cette valeur a été mesurée à partir des courbes enregistrées par le texturomètre et représentées sur la Figure 1. Les courbes correspondant à chacun des cycles sont superposées, ce qui démontre bien le caractère élastique du mélange.

### Pourcentage de l'élongation du film au point de rupture

| | |
|---|---|
| Mélange élastique | 283 % (allongement à la rupture 85 mm/lonqueur initiale 30 mm) |
| Mélange tenseur comparatif | 23% (7 mm/30 mm) |

Les courbes enregistrées par l'appareil pour chacun des deux mélanges ont été représentées à la figure 2.

### Exemple 2 : Crèmes de jour contenant un mélange élastique

On a préparé les quatre compositions de crèmes suivantes.

| INCI ou nom chimique | Exemple 2A | | Exemple 2B | | Exemple comparatif 1 |
|---|---|---|---|---|---|
| | % en poids | Phase | % en poids | Phase | |
| Eau purifiée | qsp 100 | A | qsp 100 | A | |
| Isostéaryl Isostéarate | 4 | C | 4 | C | 4 |
| Polyglycerin-3 | 3 | A | 3 | A | 3 |
| Butylène Glycol | 3 | A | 3 | A | 3 |
| Pentylène Glycol | 3 | A | 3 | A | 3 |
| Phénoxyéthanol | 0,7 | A | 0,7 | A | 0,7 |
| Decyloxazolidinone | 0,5 | C | 0,5 | C | 0,5 |
| Acrylates/C10-30 Alkyl Acrylate Crosspolymer | 0,4 | B | 0,4 | B | 0,4 |
| EDTA Tétrasodique | 0,2 | D | 0,2 | D | 0,2 |
| Hydroxyde de Sodium | < 0,1 | D | 0,1 | D | 0,1 |
| Eau purifiée | 2,8 | D | 2,8 | D | |
| Sodium Hyaluronate | < 0,1 | B | < 0,1 | B | < 0,1 |
| Eau purifiée | 5 | E | | | |
| Methyl Gluceth-20 | | | 1,8 | E | 1,8 |
| Complexe Gélifiant² | | | 7 | F | |
| Amidon¹ | 1,5 | B | 1,5 | B | |
| Glycérol | 3 | A | 3 | A | 3 |
| Polyvinyl Alcohol | 3,2 | | 3,2 | | < 0,1 |

| | | | | | |
|---|---|---|---|---|---|
| ¹ : AMIDON = Dextrine ² : Complexe gélifiant : sorbitol/cellulose gum/polyvinyl alcohol/Algin/eau, décrit dans l'exemple de la demande WO 2003/017967 | | | | | |

Les exemples 2A et 2B ont été préparés comme suit :
Mélanger la phase A sous Ystral, et la chauffer à 80°C. Ajouter l'alcool polyvinylique dans la phase A. Ajouter la phase B dans la phase A sous Ystral. Préparer la phase C au bain marie et l'ajouter à la phase A. Préparer la phase D sur bain marie et l'ajouter à la phase A. Ajouter la phase E, puis la phase F le cas échéant.

L'exemple comparatif 1 correspond à une composition contenant le mélange décrit dans la demande WO 2003/017967.

Les propriétés biomécaniques de la peau d'une personne sur laquelle on a appliqué une de ces compositions ont été évaluées avec un cutomètre de référence CUTOMETRE SEM575 de la Société Courage & Khazaka, sonde de 2 mm de diamètre. On a plus précisément mesuré la déformation résiduelle d'une zone de la peau lorsque la surface de celle-ci est aspirée dans une chambre cylindrique. La déformation a été mesurée avant et après application de chacune des trois compositions sur la peau, pour vérifier leur caractère élastique.

La méthode a consisté plus précisément à créer une dépression perpendiculaire (variant de 20 à 500 mbar) dans une petite chambre cylindrique apposée sur la peau et à mesurer au cours du temps le déplacement de la peau à l'intérieur de la chambre à partir du moment où l'on a appliqué la dépression et après rupture du vide.

L'élévation de la surface de la peau a été mesurée avec un système de mesure optique intégré, composé d'un émetteur et d'un récepteur de lumière.
On a tracé la courbe de déformation appelée « rhéogramme » de l'élongation de la peau (en mm) en fonction du temps. Le temps zéro correspondait à l'application de la dépression (Luis Rodrigues, EEMCO Guidance to the in vivo Assessment of Tensile Functional Properties of the skin, Part 2 : Instrumentation and Test Modes, Skin Pharmacol Appl Skin Physiol 2001 ; 14 : 52-67).

Les mesures ont été réalisées sur les avant-bras, à 8 cm du pli du coude, sur la face antérieure des avant-bras.
La crème a été appliquée au doigtier par la technicienne en une application unique à raison de 2,0 mg/cm². La température de réalisation de la mesure était de 22°C±2°C, et l'hygrométrie était de 50%±10%.

Dans le cadre de cette étude, la dépression a été choisie égale à 300 mbar, et on a soumis la peau à trois cycles espacés de 20 minutes de stabilisation, chaque cycle comprenant 10 séquences d'une aspiration (5 secondes) suivie d'une relaxation (1 seconde). L'application de la crème a été réalisée après le premier cycle.

On a mesuré la déformation résiduelle de la peau à l'issue de chaque cycle et constaté que la déformation résiduelle de la peau reste stable lorsque l'on applique la composition selon le procédé de l'invention, tandis que cette déformation augmente lorsque l'on applique une composition de l'art antérieur.

### Tableaux récapitulatifs des résultats :

| Déformation résiduelle | Après le premier cycle | Après le troisième cycle |
|---|---|---|
| Exemple comparatif 1 | 0.073 | 0.088 |
| Exemple 2A | 0.075 | 0.076 |
| Exemple 2B | 0.081 | 0.089 |

La rhéologie du mélange élastique, et en particulier son élasticité, présente l'avantage de ne pas varier en fonction de la température si bien que les résultats des essais qui ont été réalisés à 25°C sur un appareil de mesure en traction concordent avec les essais qui ont été réalisés une fois que le mélange a été introduit dans un produit cosmétique et appliqué sur la peau de consommatrices dont la température est de l'ordre de 33°C.

### Exemple 3 : Crème de jour comprenant un mélange élastique

La crème selon l'invention présentait la formule ci-dessous. Les pourcentages sont en poids.

### Nom INCI ou chimique

| | |
|---|---|
| AMIDON* | 0,3 |
| Alcool polyvinylique | 0,5 |
| Glycérol | 2,5 |
| COMPLEXE GELIFIANT* | 1,0 |
| Methyl methacrylate crospolymer | 1,5 |
| EDTA tétrasodique | 0,1 |
| Alkyl methyl silicone | 3,2 |
| Caprylyl glycol | 0,1 |
| Pentylène glycol | 3,0 |
| Hydroxyde de sodium | 0,1 |
| Carbomer | 0,3 |
| Gomme xanthane | 0,1 |
| Steareth-2 | 0,9 |
| Steareth-21 | 2,1 |
| Alcools gras C16-C22 | 1,6 |
| Polyéthylene | 2,6 |
| Cétéaryl isononanoate | 1,5 |
| Alkyl triglycerides | 5,2 |
| Polyisobutène hydrogéné | 4,0 |
| Diméthicone | 1,4 |
| Phospholipides | 1,0 |
| Decyloxazolidinone | 1,5 |
| Polymère gélifiant de phase grasse | 4,0 |
| Actifs cosmétiques anti-âge | 2,6 |
| Parfums | 0,3 |
| Conservateurs | 0,7 |
| Eau purifiée | qsp 100 |

| | |
|---|---|
| *AMIDON= Dextrine *COMPLEXE GELIFIANT : sorbitol/cellulose gum/polyvinyl alcohol/Algin/eau, décrit dans l'exemple de la demande WO 2003/017967. | |

La crème a été appliquée sur la peau le matin. Le dépôt formé par la composition après application était invisible du fait des propriétés de transparence du film formé par le complexe élastique de l'invention.
Les propriétés d'élasticité et de réversibilité de ce dépôt mises en évidence dans les exemples précédents ont procuré un effet lissant et tenseur tout au long de la journée malgré les mouvements du visage, et procure un aspect rebondi à la peau.

### Exemple 4 : Sérum tenseur comprenant un mélange élastique

Un sérum selon l'invention présentait la formule ci-dessous. Les pourcentages sont en poids.

### Nom INCI ou chimique

| | |
|---|---|
| AMIDON* | 0,5 |
| Alcool polyvinylique | 0,6 |
| Glycérol | 5,5 |
| COMPLEXE GELIFIANT* | 5,0 |
| Polysaccharides | 0,1 |
| Huile de jojoba estérifiée | 1,0 |
| Carboxymethyl dextran | 0,7 |
| Acrylate copolymer | 1,1 |
| EDTA tétrasodique | 0,1 |
| Caprylyl glycol | 0,3 |
| Pentaérythrityl tétraisostéarate | 4,0 |
| Hydroxyde de sodium | 0,1 |
| Ethanol | 2,0 |
| Butylène glycol 1-3 | 2,0 |
| Polyméthylsilsesquioxane | 1,0 |
| Actifs cosmétiques | 12,5 |
| Parfums | 0,3 |
| Conservateurs | 0,5 |
| Eau purifiée | qsp 100 |

| | |
|---|---|
| *AMIDON= Dextrine *COMPLEXE GELIFIANT : sorbitol/cellulose gum/polyvinyl alcohol/Algin/eau, décrit dans l'exemple de la demande WO 2003/017967. | |

Ce sérum a été appliqué sur des zones de peau présentant des signes de vieillissement cutané ou de relâchement.
Le complexe élastique a procuré à la peau un toucher particulièrement agréable, tandis que l'élasticité du film déposé après application a procuré un effet tenseur et lissant, particulièrement prolongé au cours de la journée, malgré les mouvements du visage.

## Revendications

1. Composition cosmétique contenant entre 0,2 et 90 % en poids d'un mélange apte à former un film élastique constitué :
- d'au moins un amidon gélifiant ou épaississant, susceptible de produire dans l'eau ou dans une phase aqueuse, des solutions, des suspensions ou des gels visqueux colloïdaux, et choisi parmi les hydrolysats d'amidon,
- d'au moins un polyol et
- d'au moins un polymère choisi les alcools polyvinyliques et les polymères et copolymères de vinylpyrrolidone,
dans lequel mélange, l'amidon gélifiant ou épaississant représente entre 10 et 20% en poids sec, le polymère représente de 35 à 45% en poids sec, et le polyol représente entre 35 et 45% en poids sec, par rapport au poids du mélange, la somme de ces trois pourcentages étant égale à 100.

2. Composition cosmétique selon la revendication 1, **caractérisée en ce que** l'amidon est choisi parmi les dextrines.

3. Composition cosmétique selon l'une des revendications précédentes, **caractérisé en ce que** le polyol est choisi parmi le glycérol, le sorbitol et les glycols.

4. Composition cosmétique selon l'une des revendications précédentes, **caractérisée en ce qu'**elle contient de 0,4 à 85 % en poids du mélange apte à former un film élastique.

5. Composition cosmétique selon l'une des revendications précédentes, **caractérisée en ce que** l'amidon représente de 0,05 à 5% en poids sec du poids de la composition, le polymère représente de 0,1 à 10% en poids du poids de la composition, et le polyol représente de 0,1 à 10% en poids du poids de la composition.

6. Composition cosmétique selon l'une des revendications précédentes, **caractérisée en ce que** le mélange apte à former un film élastique est constitué
- de 10 à 20 %, en poids de dextrine,
- de 35 à 45 % en poids d'alcool polyvinylique,
- de 35 à 45 %, en poids de glycérol,
ces pourcentages étant exprimés en poids sec de chacun des trois ingrédients par rapport au poids total des trois ingrédients, et la somme de ces trois pourcentages étant égale à 100.

7. Composition cosmétique selon l'une des revendications précédentes, **caractérisée en ce qu'**elle se présente sous la forme d'un gel, d'une lotion, d'un sérum, d'une suspension, d'une émulsion huile-dans-eau ou d'une émulsion eau-dans-huile.

8. Composition cosmétique selon la revendication 7, **caractérisée en ce qu'**elle se présente sous la forme d'un masque de soin anhydre, adapté à une application sur la peau du corps.

9. Procédé de soin cosmétique qui consiste à appliquer sur au moins une partie du corps, une composition cosmétique contenant de l'eau et un mélange apte à former un film élastique constitué d'au moins un amidon gélifiant ou épaississant, susceptible de produire dans l'eau ou dans une phase aqueuse, des solutions, des suspensions ou des gels visqueux colloïdaux, et choisi parmi les hydrolysats d'amidon et les dextrines, d'au moins un polymère choisi parmi les alcools polyvinyliques, les polymères et copolymères de vinylpyrrolidone et les latex, et d'au moins un polyol, le mélange étant tel que
- l'amidon représente entre 10 et 20% en poids sec,
- le polymère représente de 35 à 45% en poids sec, et
- le polyol représente entre 35 et 45% en poids sec,
par rapport au poids du mélange, la somme de ces trois pourcentages étant égale à 100.

10. Procédé selon la revendication 9, **caractérisé en ce qu'**on applique ladite composition sur au moins une zone de peau, en particulier la peau du visage ou du décolleté, présentant des signes de vieillissement ou de fatigue tels qu'une perte de fermeté, une perte d'élasticité, ou un relâchement cutané, pour obtenir un effet choisi parmi un effet lissant, un effet tenseur, un effet rebondi de la peau, ou une association de l'un de ces effets.

11. Procédé de préparation de la composition selon l'une des revendications 1 à 8, **caractérisé en ce qu'**il comprend :
- une première étape dans laquelle on prépare le mélange apte à former un film élastique par dissolution préalable du polyol dans de l'eau, puis du polymère dans l'eau, puis de l'amidon gélifiant ou épaississant dans l'eau,
- une deuxième étape dans laquelle on disperse la mélange obtenu à la première étape dans une phase aqueuse comprenant des excipients cosmétiques.

## Patentansprüche

1. Kosmetische Zusammensetzung, die zwischen 0,2 und 90 Gew.-% eines Gemischs enthält, das imstande ist, einen elastischen Film zu bilden, der ausgebildet ist aus:
- mindestens einer gelbildenden oder verdickenden Stärke, die in der Lage ist, in Wasser oder in einer Wasserphase Lösungen, Suspensionen oder kolloidale viskose Gele zu bilden und aus den Stärkehydrolysaten ausgewählt ist,
- mindestens einem Polyol und
- mindestens einem Polymer, ausgewählt aus den Polyvinylalkoholen und den Polymeren und Copolymeren von Vinylpyrrolidon,
wobei in dem Gemisch die gelbindende oder verdickende Stärke zwischen 10 und 20 % Trockengewicht darstellt, das Polymer 35 bis 45 % Trockengewicht darstellt und das Polyol zwischen 35 und 45 % Trockengewicht darstellt, in Bezug zum Gewicht des Gemischs, wobei die Summe dieser drei Prozentsätze gleich 100 ist.

2. Kosmetische Zusammensetzung nach Anspruch 1, **dadurch gekennzeichnet, dass** die Stärke aus den Dextrinen ausgewählt ist.

3. Kosmetische Zusammensetzung nach einem der vorangehenden Ansprüche, **dadurch gekennzeichnet, dass** das Polyol aus dem Glycerol, dem Sorbitol und den Glycolen ausgewählt ist.

4. Kosmetische Zusammensetzung nach einem der vorangehenden Ansprüche, **dadurch gekennzeichnet, dass** sie 0,4 bis 85 Gew.-% des Gemischs enthält, das imstande ist, einen elastischen Film zu bilden.

5. Kosmetische Zusammensetzung nach einem der vorangehenden Ansprüche, **dadurch gekennzeichnet, dass** die Stärke 0,05 bis 5 % Trockengewicht des Gewichts der Zusammensetzung darstellt, das Polymer 0,1 bis 10 Gew.-% des Gewichts der Zusammensetzung darstellt und das Polyol 0,1 bis 10 Gew.-% des Gewichts der Zusammensetzung darstellt.

6. Kosmetische Zusammensetzung nach einem der vorangehenden Ansprüche, **dadurch gekennzeichnet, dass** das Gemischs, das imstande ist, einen elastischen Film zu bilden, ausgebildet ist aus
- von 10 bis 20 Gew.-% Dextrin,
- von 35 bis 45 Gew.-% Polyvinylalkohol,
- von 35 bis 45 Gew.-% Glycerol,
wobei diese Prozentsätze in Trockengewicht jeder der drei Inhaltsstoffe in Bezug zum Gesamtgewicht der drei Inhaltsstoffe ausgedrückt sind und die Summe dieser drei Prozentsätze gleich 100 ist.

7. Kosmetische Zusammensetzung nach einem der vorangehenden Ansprüche, **dadurch gekennzeichnet, dass** sie in Form eines Gels, einer Lotion, eines Serums, einer Suspension, einer Öl-in-Wasser-Emulsion oder einer Wasser-in-ÖI-Emulsion vorliegt.

8. Kosmetische Zusammensetzung nach Anspruch 7, **dadurch gekennzeichnet, dass** sie in Form einer wasserfreien Pflegemaske vorliegt, die für ein Auftragen auf die Haut des Körpers geeignet ist.

9. Kosmetisches Pflegeverfahren, das darin besteht, auf mindestens einen Teil des Körpers eine kosmetische Zusammensetzung aufzutragen, die Wasser und ein Gemisch enthält, das imstande ist, einen elastischen Film zu bilden, gebildet von mindestens einer gelbildenden oder verdickenden Stärke, die in der Lage ist, in Wasser oder in einer Wasserphase Lösungen, Suspensionen oder kolloidale viskose Gele zu bilden und aus den Stärkehydrolysaten und den Dextrinen ausgewählt ist, mindestens einem Polymer, ausgewählt aus den Polyvinylalkoholen, den Polymeren und Copolymeren von Vinylpyrrolidon und den Latexen, und mindestens einem Polyol, wobei das Gemisch derart ist, dass
- die Stärke zwischen 10 und 20 % Trockengewicht darstellt,
- das Polymer 35 bis 45 % Trockengewicht darstellt, und
- das Polyol zwischen 35 und 45 % Trockengewicht darstellt
in Bezug zum Gewicht des Gemischs, wobei die Summe dieser drei Prozentsätze gleich 100 ist.

10. Verfahren nach Anspruch 9, **dadurch gekennzeichnet, dass** die Zusammensetzung auf mindestens einen Bereich der Haut, insbesondere auf die Haut des Gesichts oder des Dekolletees, aufgetragen wird, die Zeichen von Alterung oder von Erschlaffung wie einen Verlust von Festigkeit, einen Verlust von Elastizität oder eine Hauterschlaffung aufweist, um eine Wirkung, ausgewählt aus einer glättenden Wirkung, einer straffenden Wirkung, einer aufpolsternde Wirkung der Haut oder eine Kombination einer dieser Wirkungen, zu erhalten.

11. Verfahren zur Herstellung der Zusammensetzung nach einem der Ansprüche 1 bis 8, **dadurch gekennzeichnet, dass** es umfasst:
- einen ersten Schritt, bei dem das Gemisch, das imstande ist, einen elastischen Film zu bilden, durch vorheriges Auflösen des Polyols in Wasser, dann des Polymers in Wasser, dann der gelbildenden oder verdickenden Stärke in Wasser, hergestellt wird,
- einen zweiten Schritt, bei dem das im ersten Schritt erhaltene Gemisch in einer Wasserphase, die kosmetische Hilfsstoffe umfasst, verteilt wird.

## Claims

1. A cosmetic composition containing between 0.2 and 90 wt% of a mixture able to form an elastic film consisting of:
- at least one starch with gelling or thickening action, likely to produce in water or in an aqueous phase, viscous colloidal solutions, suspensions or gels, and selected from starch hydrolyzates,
- at least one polyol and
- at least one polymer selected from polyvinyl alcohols and vinylpyrrolidone polymers and copolymers latex, in which mixture, the starch with gelling or thickening action represents between 10 and 20 wt% dry matter, the polymer represents from 35 to 45 wt% dry matter, and the polyol represents between 35 and 45 wt% dry matter, relative to the weight of the mixture, the sum of these three percentages being equal to 100.

2. The cosmetic composition as claimed in claim 1, **characterized in that** the starch is selected from the dextrins.

3. The cosmetic composition as claimed in one of the preceding claims, **characterized in that** the polyol is selected from glycerol, sorbitol and the glycols.

4. The cosmetic composition as claimed in one of the preceding claims, **characterized in that** it contains from 0.4 to 85 wt% of the mixture able to form an elastic film.

5. The cosmetic composition as claimed in one of the preceding claims, **characterized in that** the starch represents from 0.05 to 5 wt% dry matter of the weight of the composition, the polymer represents from 0.1 to 10 wt% of the weight of the composition, and the polyol represents from 0.1 to 10 wt% of the weight of the composition.

6. The cosmetic composition as claimed in one of the preceding claims, **characterized in that** the mixture able to form an elastic film consists of
- 10 to 20 wt% of dextrin,
- 35 to 45 wt% of polyvinyl alcohol,
- 35 to 45 wt% of glycerol,
these percentages being expressed in dry weight of each of the three ingredients relative to the total weight of the three ingredients, with the sum of these three percentages being equal to 100.

7. The cosmetic composition as claimed in one of the preceding claims, **characterized in that** it is in the form of a gel, a lotion, a serum, a suspension, an oil-in-water emulsion or a water-in-oil emulsion.

8. The cosmetic composition as claimed in claim 7, **characterized in that** it is in the form of an anhydrous care mask, suitable for application on the skin of the body.

9. A method of cosmetic care that consists of applying, on at least one part of the body, a cosmetic composition containing water and a mixture able to form an elastic film consisting of at least one starch with gelling or thickening action, likely to produce in water or in an aqueous phase, viscous colloidal solutions, suspensions or gels, and selected from starch hydrolyzates or dextrins, at least one polymer selected from polyvinyl alcohols, vinylpyrrolidone polymers and copolymers and latex, and at least one polyol, the mixture being such that
- the starch represents between 10 and 20 wt% dry matter,
- the polymer represents from 35 to 45 wt% dry matter, and
- the polyol represents between 35 and 45 wt% dry matter,
relative to the weight of the mixture, the sum of these three percentages being equal to 100.

10. The method as claimed in claim 9, **characterized in that** said composition is applied on at least one area of skin, in particular the skin of the face or cleavage, showing signs of aging or tiredness such as loss of firmness, loss of elasticity, or skin sag, to obtain an effect selected from a smoothing effect, a lifting effect, an effect of plumpness of the skin, or a combination of two or more of these effects.

11. A method of preparing the composition as claimed in one of claims 1 to 8, **characterized in that** it comprises:
- a first step in which the mixture able to form an elastic film is prepared by first dissolving the polyol in water, then the polymer in water, then the starch with gelling or thickening action in water,
- a second step in which the mixture obtained in the first step is dispersed in an aqueous phase comprising cosmetic excipients.
